# EUROPÄISCHE PATENTSCHRIFT

(11) **EP 0 436 786 B1**
(45) Veröffentlichungstag und Bekanntmachung des Hinweises auf die Patenterteilung: **08.06.1994**
(21) Anmeldenummer: 90120198.8
(22) Anmeldetag: 22.10.1990
(51) Int. Cl.: B30B 11/22, B29B 9/06

(54) **Verfahren und Vorrichtung zum Herstellen von Extrudatteilchen**
Process and apparatus for extruding pellets
Procédé et dispositif pour la fabrication de boulettes extrudées

(30) Priorität: 10.01.1990 DE 4000571
(43) Veröffentlichungstag der Anmeldung: 17.07.1991
(73) Patentinhaber: Hüttlin Coating-Technik GmbH, D-79585 Steinen (DE)
(72) Erfinder: Hüttlin, Herbert, D-79585 Steinen (DE)
(74) Vertreter: Weller, Wolfgang, Dr.rer.nat.

(56) Entgegenhaltungen:
- CH-A- 453 664
- DE-A- 2 200 114
- FR-A- 2 039 455
- US-A- 3 213 170
- US-A- 3 759 642
- US-A- 3 846 529
- PATENT ABSTRACTS OF JAPAN vol. 13, no. 588 (M-912)(3936) 25. Dezember 1989 & JP-A-1 247 112 (HITACHI LTD. ) 3. Oktober 1989
- PATENT ABSTRACTS OF JAPAN vol. 6, no. 23 (M-111)(901) 10. Februar 1982 & JP-A-56 142 014 (NIPPON SEIKOSHO K.K. ) 6. November 1981

## Beschreibung

Die Erfindung betrifft ein Verfahren und eine Vorrichtung nach dem Oberbegriff des Anspruchs 1 bzw. des Anspruchs 6.

Ein Verfahren und eine Vorrichtung dieser Gattung sind aus den Dokumenten US-A-3,846,529 bzw. der US-A-3,759,642 bekannt. Dabei wird eine teigige Masse von einem Schneckenförderer durch eine senkrechte Düsenplatte hindurch waagerecht in eine Kammer extrudiert, in der ein allgemeiner Gasstrom waagerecht gegen die Mitte der Düsenplatte gerichtet und in einstellbarem Abstand von ihr durch einen trompetenartigen Ablenkkörper radial umgelenkt wird. In die Kammer münden außerdem mehrere parallel zur Düsenplatte nach oben gerichtete Druckgasdüsen, deren Lage einstellbar ist. Die Extrudatteilchen werden vom allgemeinen Gasstrom und von aus den Druckgasdüsen austretenden Druckgasstrahlen abgeschert.

Bei diesem bekannten Verfahren und der dafür verwendeten Vorrichtung besteht die Gefahr, daß die Extrudatteilchen auf ihrer Leeseite feucht bleiben und deshalb nach dem Herabfallen mit anderen Extrudatteilchen verkleben. Stellt man hingegen den allgemeinen Gasstrom und/oder die Druckgasstrahlen schwach ein, damit die Extrudatteilchen gleichmäßig trocknen, dann besteht die Gefahr, daß sie nicht rechtzeitig brechen und somit ungleichmäßig lang werden.

Aus dem Dokument JP-A-56 142 014 ist es ferner bekannt, geschmolzenes Kunstharz durch einen Extrusionskanal zu extrudieren, in dessen Endbereich ein Fluidkanal unter einem spitzen Winkel mündet. In diesen wird stoßweise ein Fluid unter Druck eingeleitet, das das Extrudat in einzelne Pellets zerschneidet. Auch hier besteht die Gefahr, daß die Pellets anschließend miteinander verkleben, da sie vom Fluidstrahl nur einseitig gekühlt werden können.

Der Erfindung liegt deshalb die Aufgabe zugrunde, ein Verfahren zum Herstellen von Extrudatteilchen derart weiterzubilden und eine Vorrichtung zum Durchführen des Verfahrens derart zu gestalten, daß die Gefahr des Verklebens der Extrudatteilchen sicher vermieden wird.

Die Aufgabe ist, soweit sie ein Verfahren betrifft, mit den Merkmalen des Anspruchs 1 und, soweit sie eine Vorrichtung betrifft, mit den Merkmalen des Anspruchs 6 gelöst.

Vorteilhafte Weisterbildungen der Erfindung ergeben sich aus den Unteransprüchen.

Ein Ausführungsbeispiel einer erfindungsgemäßen Vorrichtung wird im folgenden anhand schematischer Zeichnunen mit weiteren Einzelheiten erläutert. Es zeigen:
- Fig. 1: einen Querschnitt durch eine Vorrichtung zum Herstellen von Extrudatteilchen aus einer teigigen Masse,
- Fig. 2: die zugehörige, teilweise als Schnitt II-II in Fig. 1 gezeichnete Seitenansicht,
- Fig. 3: einen stark vergrößerten Ausschnitt aus Fig. 1,
- Fig. 4: die Ansicht in Richtung des Pfeils IV in Fig. 3,
- Fig. 5: einen der Fig. 3 entsprechenden Ausschnitt mit abgewandelten Einzelheiten und
- Fig. 6: die Ansicht in Richtung des Pfeils VI in Fig. 5.

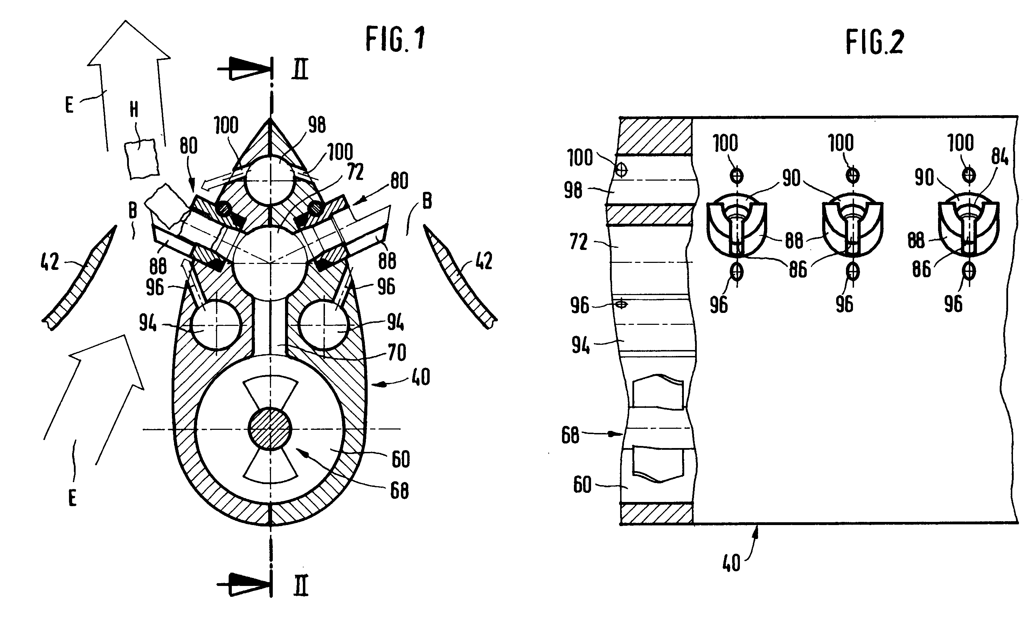

Gemäß Fig. 1 sind in einer Prozeßkammer 12 eine Lanze 40 und ein Paar Leitflügel 42 symmetrisch in bezug auf eine senkrechte Mittelebene A angeordnet. Die Lanze 40 hat ein zur Mittelebene A symmetrisches, tropfenförmiges Profil, das unten abgerundet und oben spitz ist. Die beiden Leitflügel 42 sind parallel zur Lanze 40 angeordnet und bilden mit ihr je einen Spalt B. Durch die Spalte B kann Prozeßluft in Form eines allgemeinen Gasstromes E um das Profil der Lanze 40 herum nach oben strömen und oberhalb von ihr ein Fließbett bilden.

Die Lanzen 40 haben je einen in ihrer Längsrichtung verlaufenden Förderkanal 60 von kreisförmigem Querschnitt, der gleichachsig mit dem ebenfalls kreisbogenförmigen unteren Teil des Lanzenprofils angeordnet ist. An die beiden Enden des Förderkanals 60 ist je eine Pumpe angeschlossen, beispielsweise eine handelsübliche Exzenterschneckenpumpe, die von einem Elektromotor angetrieben wird und eine zu extrudierende teigige Masse in den Förderkanal 60 preßt.

Der Förderkanal 60 ist durch einen Längsschlitz 70 mit einem Verteilkanal 72 verbunden, der sich ebenfalls in Längsrichtung der Lanze 40 erstreckt, und von dem in gleichmäßigen Abständen Bohrungen 74 schräg nach oben zur Oberfläche der Lanze 40 führen. Die Bohrungen 74 haben je eine Schulter 76, an der sie sich nach außen erweitern, und an der ein Dichtungsring 78 anliegt. In den erweiterten äußeren Bereich der Bohrungen 74 ist je eine Extrusionsdüse 80 bis zur Schulter 76 hin eingeschoben.

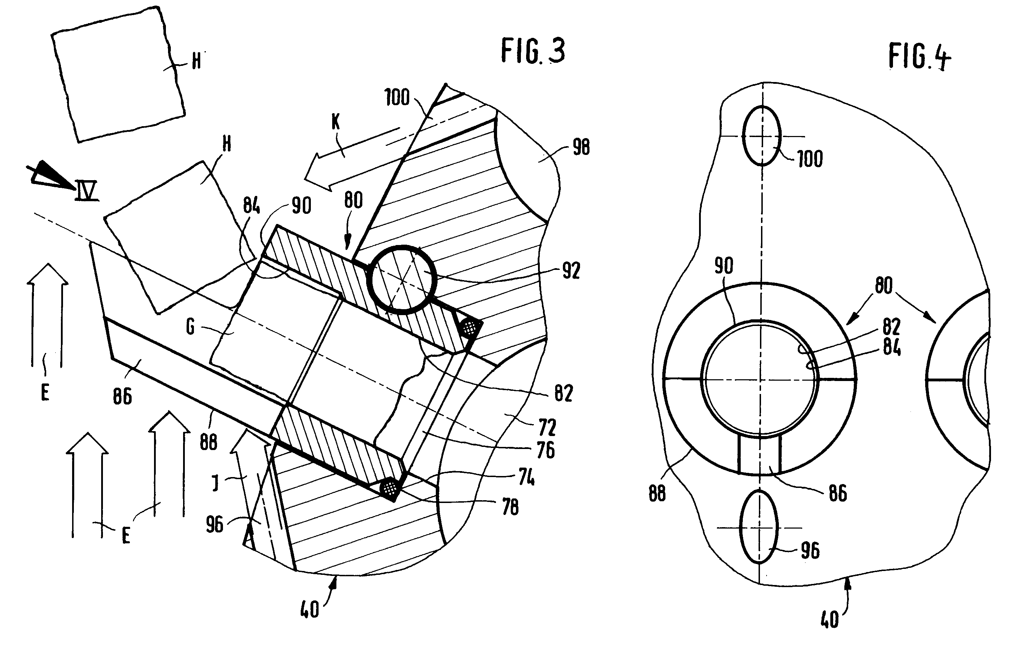

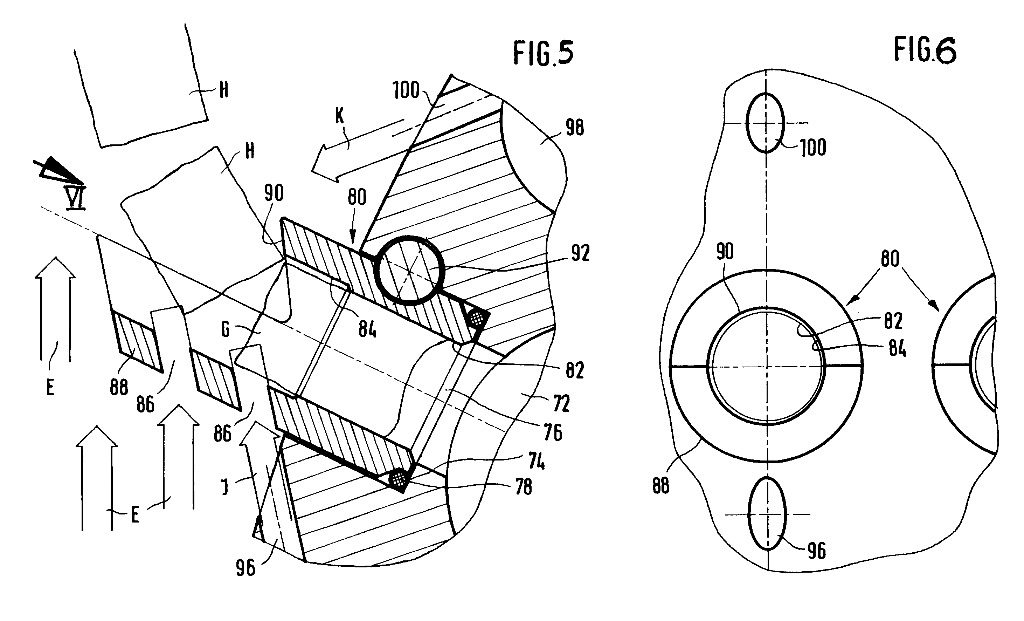

Jede Extrusionsdüse 80, von der eine Ausführungsform in Fig. 3 und 4 sowie eine andere Ausführungsform in Fig. 5 und 6 dargestellt ist, hat einen axial inneren engeren Bereich 82, der den Durchmesser eines in der Extrusionsdüse 80 entstehenden Strangs G aus teigiger Masse bestimmt, und einen axial äußeren erweiterten Bereich 84, der durch mindestens einen Durchbruch 86 hindurch von außen belüftbar ist, um die Trocknung des Strangs G zu fördern. Gemäß Fig. 3 und 4 ist als Durchbruch 86 ein axialer Schlitz vorgesehen, der bezogen auf den allgemeinen Gasstrom E an der Luvseite der Extrusionsdüse 80 angeordnet ist und sich vom Anfang des erweiterten Bereichs 84 bis zum freien Ende der Extrusionsdüse 80 erstreckt. Gemäß Fig. 5 und 6 sind, ebenfalls an der Luvseite der Extrusionsdüse 80, zwei Durchbrüche 86 in Form paralleler schräger Schlitze angeordnet.

Bei beiden in Fig. 3 und 4 einerseits sowie in Fig. 5 und 6 andererseits dargestellten Ausführungsformen der Extrusionsdüse 80 ist deren in bezug auf den allgemeinen Gasstrom E in Lee liegende Hälfte im axial äußeren Teil des erweiterten Bereichs 84 abgetragen, beispielsweise abgefräst, so daß auf der Luvseite ein halbrohrförmiger Schild 88 übrigbleibt und auf der Leeseite eine Abrißkante 90 gebildet ist, die gemäß Fig. 3 und 4 in einer zur Längsachse der Extrusionsdüse 80 normalen Ebene liegt, gemäß Fig. 5 und 6 hingegen parallel zu den schrägen Durchbrüchen 86 angeordnet ist.

In jeder Lanze 40 sind zwei Riegel 92 angeordnet, die sich auf je einer Seite der Mittelebene A in Längsrichtung der Lanze 40 erstrecken und die in der betreffenden Hälfte der Lanze 40 eingeschobenen Extrusionsdüsen 80 gegen axiale Verschiebung sowie gegen Drehen sichern.

Jede Lanze 40 enthält ferner zwei zu ihrem Förderkanal 60 parallele untere Druckgaskanäle 94, von denen Druckgasdüsen 96 derart ausgehen, daß auf den oder einen Durchbruch 86 jeder Extrusionsdüse 80 eine Druckgasdüse 96 gerichtet ist. Die Druckgaskanäle 94 werden über Elektromagnetventile bekannter Bauart intermittierend mit Druckluft versorgt, die getrocknet und vorgewärmt sein kann. Somit geben die unteren Druckgasdüsen 96 stoßweise Druckluft auf den Mündungsbereich der zugehörigen Extrusionsdüse 80 ab. Die Druckluft wirkt in einem scharf gebündelten Strahl J durch den bzw. einen Durchbruch in der zugehörigen Extrusionsdüse 80 stoßweise auf den Strang G ein, so daß von diesem bei jedem Druckluftstoß ein Extrudatteilchen H abbricht. In den Zeitintervallen zwischen je zwei Druckluftstößen kann ein schwächerer Druckluftstrom aus den Druckgasdüsen 96 austreten.

Im oberen Bereich jeder Lanze 40 ist, ebenfalls parallel zum Förderkanal 60, ein weiterer Druckgaskanal 98 angeordnet, der kontinuierlich und/oder stoßweise im Wechsel mit den Druckgaskanälen 94 mit warmer, trockener Druckluft versorgt wird. Vom oberen Druckgaskanal 98 gehen Druckgasdüsen 100 aus, die von oben her unter einem spitzen Winkel gegen den Mündungsbereich je einer Extrusionsdüse 80 gerichtet sind. Das sich darin bildende Extrudatteilchen H wird somit auch auf der Seite, die bezüglich des allgemeinen Gasstroms E die Leeseite ist, von einem Warmluftstrahl K getrocknet und durch dessen Druck daran gehindert, vorzeitig abzubrechen, ehe sein freies Ende das Ende des Schildes 88 erreicht hat.

Der Winkel, den jede der Druckgasdüsen 96 und 100 mit der Achsrichtung der zugehörigen Extrusionsdüse 80 einschließt, beträgt vorzugsweise ungefähr 30° bis 50°, so daß die von den Druckgasdüsen 96 und 100 ausgehenden Druckgasstrahlen J und K auf den Strang G Kraftimpulse ausüben, die eine Komponente in Extrusionsrichtung haben.

Die Extrusionsdüsen 80 bestehen beispielsweise aus Edelmetall oder Kunststoff und sind austauschbar, beispielsweise gegen Extrusionsdüsen mit einem engen Bereich 82 von größerem oder kleinerem Durchmesser und/oder mit einem längeren oder kürzeren Schild 88. Auf diese Weise lassen sich Durchmesser und Länge der entstehenden Extrudatteilchen H verändern, wobei die Frequenz der durch die Druckgasdüsen 96 zugeführten Druckluftstöße an die Extrusionsgeschwindigkeit des Strangs G und die gewünschte Länge der Extrudatteilchen H anzupassen ist.

Die abgebrochenen Extrudatteilchen H werden vom allgemeinen Gasstrom E nach oben getragen, wobei sich ein Fließbett oder Wirbelbett bildet, in dem die Extrudatteilchen H weiter getrocknet werden.

## Patentansprüche

1. Verfahren zum Herstellen von Extrudatteilchen (H), insbes. als Vorstufe für pharmazeutische Pellets, bei dem
- eine teigige Masse in Form eines Strangs (G) quer zu einem allgemeinen Gasstrom (E) in diesen hinein extrudiert wird, und
- ein Vortrocknen und Brechen des Stangs (G) durch mindestens einen auf den Strang (G) gerichteten Druckgasstrahl (J) unterstützt wird,
dadurch **gekennzeichnet**, daß
- der Druckgasstrahl (J) ungefähr in gleicher Richtung wie der allgemeine Gasstrom (E) stoßweise auf den Strang (G) gerichtet wird und
- ein ebenfalls scharf gebündelter zusätzlicher Gasstrahl (K) auf die Leeseite des Strangs (G) gerichtet wird.

2. Verfahren nach Anspruch 1,
dadurch **gekennzeichnet**, daß der zusätzliche Gasstrahl (K) im Wechsel mit dem Druckgasstrahl (J) stoßweise auf den Strang (G) gerichtet wird.

3. Verfahren nach Anspruch 1 oder 2,
dadurch **gekennzeichnet**, daß der Druckgasstrahl (J) ständig mit einer Grundleistung und zusätzlich intervallweise mit einer Spitzenleistung betrieben wird.

4. Verfahren nach einem der Ansprüche 1 bis 3,
dadurch **gekennzeichnet**, daß der in den allgemeinen Gasstrom (E) eintretende Strang (G) an seiner Luvseite auf einer begrenzten Länge gegen den allgemeinen Gasstrom (E) abgeschirmt wird, während er auf seiner Leeseite schon freiliegt.

5. Verfahren nach einem der Ansprüche 1 bis 4,
dadurch **gekennzeichnet**, daß der allgemeine Gasstrom (E) aufwärtsgerichtet ist und ein Wirbelbett (F) bildet, in dem die Extrudatteilchen (H) unmittelbar nach ihrem Abbrechen vom Strang (G) weiterbehandelt werden.

6. Vorrichtung zum Durchführen des Verfahrens nach einem der Ansprüche 1 bis 5 mit
- einer Prozeßkammer (12), in der sich ein allgemeiner Gasstrom (E) erzeugen läßt,
- quer zum allgemeinen Gasstrom (E) in die Prozeßkammer (12) mündenden Extrusionsdüsen (80) zum Extrudieren teigiger Masse und
- Druckgasdüsen (96), die ebenfalls in die Prozeßkammer (12) münden,
dadurch **gekennzeichnet**, daß
- die Extrusionsdüsen (80) und Druckgasdüsen (96) an einer hohlen Lanze (40) angeordnet sind, die sich in der Prozeßkammer (12) quer zum allgemeinen Gasstrom (E) erstreckt,
- die Druckgasdüsen (96) unter einem spitzen Winkel zur Extrusionsrichtung und zur Richtung des allgemeinen Gasstroms (E) luvseitig auf den Mündungsbereich je einer Extrusionsdüse (80) gerichtet sind, und
- an der Lanze (40) zusätzliche Druckgasdüsen (100) angeordnet sind, die ebenfalls unter einem spitzen Winkel, jedoch bezüglich des allgemeinen Gasstroms (E) leeseitig, gegen den Mündungsbereich je einer Extrusionsdüse (80) gerichtet sind.

7. Vorrichtung nach Anspruch 6,
dadurch **gekennzeichnet**, daß die Extrusionsdüsen (80) je einen den Querschnitt des Strangs (G) bestimmenden engen Bereich (82) und einen sich daran anschließenden erweiterten Bereich (84) aufweisen, der durch mindestens einen Durchbruch (86) zur zugehörigen Druckgasdüse (96) hin geöffnet ist.

8. Vorrichtung nach Anspruch 6 oder 7,
dadurch **gekennzeichnet**, daß die Extrusionsdüsen (80) auf ihrer Luvseite, bezogen auf den allgemeinen Gasstrom (E), je einen Schild (88) aufweisen, der den extrudierten Strang (G) auf einer bestimmten Länge gegen die zugehörige Druckgasdüse (96) abschirmt.

9. Vorrichtung nach einem der Ansprüche 6 bis 8,
dadurch **gekennzeichnet**, daß die Lanze (40) ein zu einer senkrechten Mttelebene (A) symmetrisches Profil hat und vom allgemeinen Gasstrom (E) von unten nach oben symmetrisch umströmt ist.

10. Vorrichtung nach Anspruch 9,
dadurch **gekennzeichnet**, daß beiderseits der Lanze (40), parallel und symmetrisch zu ihr, Leitflügel (42, 44) angeordnet sind, die den allgemeinen Gasstrom (E) mindestens annähernd im rechten Winkel zu den Extrusionsdüsen (80) leiten.

## Claims

1. Process for extruding pellets formed from an extruded material, particularly designed to serve as preforms for pharmaceutical tablets, wherein
- a pasty material in the shape of a strand (G) is extruded in a manner to enter into a general gas flow (E) in a transverse direction thereto, and
- a preliminary drying and breaking up of the strand (G) is facilitated by at least one pressurized gas jet (J) directed against the strand (G),
**characterized** in that
- the pressurized gas jet (J) is directed against the strand (G) in approximately the same direction as is the general gas flow (E) and in an intermittent manner, and
- an additional gas jet (K), which is also sharply focussed, is directed against the leeward side of the strand (G).

2. Process according to claim 1,
**characterized** in that the additional gas jet (K) is directed against the strand (G) in alternation with the pressurized gas jet (J) and in an intermittent manner.

3. Process according to claim 1 or 2,
**characterized** in that the pressurized gas jet (J) is operated with a permanent basic output and additionally with an intermittent maximum output.

4. Process according to any of claims 1 to 3,
**characterized** in that the strand (G) which enters into the general gas flow (E) is protected on its windward side from the general gas flow (E) over a limited distance while it is already exposed on its leeward side.

5. Process according to any of claims 1 to 4,
**characterized** in that the general gas flow (E) is upwardly directed and produces a fluidized bed (F) in which the pellets of extruded material are subjected to a further process immediately after their separation from the strand (G).

6. Apparatus for implementing the process according to any of claims 1 to 5, comprising
- a processing chamber (12) in which a general gas flow (E) is capable of being produced,
- extrusion dies (80) for extruding a pasty material, opening into the processing chamber (12) in a direction transverse to the general gas flow (E), and
- pressurized gas nozzles (96) which also open into the processing chamber (12),
**characterized** in that
- the extrusion dies (80) and the pressurized gas nozzles (96) are arranged on a hollow lance (40) which is extending inside the processing chamber (12) in a direction transverse to the general gas flow (E),
- the pressurized gas nozzles (96) are directed against the windward side of the mouth region of each extrusion die (80), forming an acute angle with the extrusion direction and with the direction of the general gas flow (E), and
- further pressurized gas nozzles (100) are arranged on the lance (40) which are directed against the mouth region of each extrusion die (80), also forming an acute angle, but acting on the leeward side with respect to the general gas flow (E).

7. Apparatus according to claim 6,
**characterized** in that the extrusion dies (80) each have a narrow portion (82) which is determining for the cross-section of the strand (G) and an adjacent enlarged portion (84) which opens towards the associated pressurized gas nozzle (96) through at least one opening (86).

8. Apparatus according to claim 6 or 7,
**characterized** in that the extrusion dies (80) are each provided on their windward side, when considering the general gas flow (E), with a shield (88) which protects the extruded strand (G) over a certain distance from the associated pressurized gas nozzle (96).

9. Apparatus according to any of claims 6 to 8,
**characterized** in that the lance (40) has a symmetrical profile centered to a vertical central plane (A) and has the general gas flow (E) flow around it in a symmetrical manner from below upward.

10. Apparatus according to claim 9,
**characterized** in that on both sides of the lance (40) guiding vanes (42, 44) are arranged in parallel and symmetrical relationship, said vanes directing the general gas flow (E) in a direction substantially normal to the extrusion dies (80).

## Revendications

1. Procédé de fabrication de boulettes (H) en masse extrudée, plus particulièrement destinées à servir de préformes pour tablettes pharmaceutiques, procédé dans lequel
- une masse pâteuse sous la forme d'un boudin (G) est extrudée dans une direction transversale par rapport à un courant de gaz général (E) et de façon à entrer dans ce dernier, et
- un jet de gaz comprimé (J), au moins, qui est dirigé vers le boudin (G), contribue à un séchage préliminaire et une rupture du boudin (G),
**caractérisé** en ce que
- le jet de gaz comprimé (J) est dirigé vers le boudin (G), sensiblement dans la même direction que le courant de gaz général (E), de façon intermittente, et
- qu'un jet de gaz additionnel (K), lui aussi fortement concentré, est dirigé vers le côté non exposé au courant (E).

2. Procédé selon la revendication 1,
**caractérisé** en ce que le jet de gaz additionnel (K) est dirigé vers le boudin (G) de façon intermittente et en alternance avec le jet de gaz comprimé (J).

3. Procédé selon la revendication 1 ou 2,
**caractérisé** en ce que le jet de gaz comprimé (J) est opéré avec un débit de base permanent et, additionnellement, avec un débit maximal intermittent.

4. Procédé selon une quelconque des revendications 1 à 3,
**caractérisé** en ce que, lorsqu'il entre dans le courant de gaz général (E), le boudin (G) est protégé, sur son côté faisant face au courant (E), du courant de gaz général (E), sur une distance limitée, alors qu'il est déjà exposé sur son côté opposé par rapport au courant (E).

5. Procédé selon une quelconque des revendications 1 à 4,
**caractérisé** en ce que le courant de gaz général (E) est dirigé vers le haut et forme un lit fluidisé (F) dans lequel les boulettes (H) en masse extrudée sont soumises à un traitement supplémentaire immédiatement après leur détachement du boudin (G).

6. Dispositif pour la mise en oeuvre du procédé selon une quelconque des revendications 1 à 5, ledit dispositif comprenant
- une chambre de traitement (12), dans laquelle un courant de gaz général (G) est capable d'être généré,
- des filières d'extrusion (80) destinées à extruder une masse pâteuse et débouchant dans la chambre de traitement (12) en direction transversale par rapport au courant de gaz général (E), et
- des buses à gaz comprimé (96) qui débouchent aussi dans la chambre de traitement (12),
**caractérisé** en ce que
- les filières d'extrusion (80) et les buses à gaz comprimé (96) sont disposées sur une lance creuse (40) qui s'étend dans la chambre de traitement (12) en direction transversale par rapport au courant de gaz général (E),
- les buses à gaz comprimé (96) sont dirigées, sous un angle aigu par rapport à la direction d'extrusion et à la direction du courant de gaz général (E), vers le côté faisant face au courant (E) de la région où chaque filière d'extrusion (80) débouche, et
- des buses à gaz comprimé (100) additionnelles sont disposées sur la lance (40), ces buses étant dirigées, également en formant un angle aigu, vers la région où débouche chaque filière d'extrusion (80), mais sur le côté opposé par rapport au courant de gaz général (E).

7. Dispositif selon la revendication 6,
**caractérisé** en ce que les filières d'extrusion (80) ont chacune une partie restreinte (82) qui détermine la section droite du boudin (G), et une partie élargie adjacente (84) qui s'ouvre sur la buse à gaz comprimé (96) qui lui est associée par l'intermédiaire d'un ajour (86), au moins.

8. Dispositif selon la revendication 6 ou 7,
**caractérisé** en ce que les filières d'extrusion (80) comportent chacune sur leur côté faisant face au courant de gaz général (E) un écran (88) qui protège le boudin extrudé (G) sur une distance déterminée contre la buse à gaz comprimé (96) associée.

9. Dispositif selon une quelconque des revendications 6 à 8,
**caractérisé** en ce que la lance (40) possède un profil symétrique par rapport à un plan médian vertical (A), et que le courant de gaz général (E) s'écoule autour de la lance, de bas en haut, de façon symétrique.

10. Dispositif selon la revendication 9,
**caractérisé** en ce que des deux côtés de la lance (40) des pales de guidage (42, 44) sont disposées de façon parallèle et symétrique par rapport à elle, lesdites pales dirigeant le courant de gaz général (E) sous un angle sensiblement normal aux filières d'extrusion (80).
